# EUROPEAN PATENT SPECIFICATION

(11) **EP 3 003 256 B1**
(45) Date of publication and mention of the grant of the patent: **10.10.2018**
(21) Application number: 14726622.5
(22) Date of filing: 28.05.2014
(51) Int. Cl.: A61Q 5/02, A61K 8/87, A61K 8/44, A61K 8/46

(54) **COSMETIC COMPOSITION COMPRISING NONIONIC ASSOCIATIVE POLYMERS AND CARBOXYLATE ANIONIC SURFACTANTS, AND COSMETIC TREATMENT PROCESS**
KOSMETISCHE ZUSAMMENSETZUNG MIT NICHTIONISCHEN ASSOZIATIVEN POLYMEREN UND ANIONISCHEN CARBOXYLATTENSIDEN SOWIE KOSMETISCHES BEHANDLUNGSVERFAHREN DAMIT
COMPOSITION COSMÉTIQUE COMPRENANT DES POLYMÈRES ASSOCIATIFS NON IONIQUES ET DES TENSIOACTIFS ANIONIQUES DE TYPE CARBOXYLATE, ET UN PROCÉDÉ DE TRAITEMENT COSMÉTIQUE

(30) Priority: 03.06.2013 FR 1355041
(43) Date of publication of application: 13.04.2016
(73) Proprietor: L'Oréal, 75008 Paris (FR)
(72) Inventor: D'ARRAS, Marie-Florence, F-92110 Clichy (FR); MATHONNEAU, Estelle, 93400 Saint-Ouen (FR)
(74) Representative: Dodin, Catherine
(86) International application number: PCT/EP2014/061042
(87) International publication number: WO 2014/195202

(56) References cited:
- WO-A2-2011/073564
- FR-A1- 2 935 267

## Description

The present invention relates to a cosmetic composition, according to claim 1 and also to a cosmetic treatment process using the said composition.
Hair has a tendency to lose some of its qualities due to the action of factors especially such as natural regreasing, sweat, the removal of squamae, pollution or humidity. The visual appearance and the feel of the hair can thus be damaged. Regreasing, for example, makes the hair lank, which then has a tendency to clump together. The hair may be increasingly difficult to style, and may have an unpleasant greasy sheen or an unpleasant waxy feel.
It is known practice to cleanse the hair with shampoos, which are generally aqueous compositions containing large amounts of surfactants, which are generally anionic surfactants, alone or in combination with amphoteric and/or nonionic surfactants. The total amounts of surfactants used usually exceed 10% by weight of active material relative to the total weight of the cosmetic composition.
These shampoos based on large amounts of anionic surfactants may cause discomfort such as stinging of the scalp or of the eyes when they come into contact with the shampoo.
Moreover, gradually in the course of their applications, these surfactants may impair the cosmetic properties of the hair, thus leading to the need also to use conditioning agents such as cationic polymers, silicones or non-silicone oils.
In addition, the rinsing of cosmetic compositions with a high content of surfactants may often be long.
Finally, to avoid running on application and especially running into the eyes, shampoos should generally be thickened; but their thickening may pose problems of stability of the composition.
In order to overcome these various problems, it has been proposed, for example by patent application FR 2 935 267, to add associative polymers to shampoo compositions, which makes it possible to reduce their content of standard surfactants. At and above a certain concentration, these associative polymers have sufficient detergent power to enable cleansing of the hair in the presence of very small amounts of surfactants, or even in the absence of these surfactants.
However, although they afford detergency similar to that obtained with a standard shampoo, the compositions thus obtained still have insufficient foaming nature; in addition, the cosmetic properties imparted to the hair are still not entirely satisfactory, in particular on dry hair.

The aim of the present invention is to propose cosmetic hair compositions that overcome these drawbacks, and especially that are capable of generating an adequate foam, both in quality and quantity, and that give the hair satisfactory cosmetic properties, most particularly on dry hair.

One subject of the invention is thus a non-colouring cosmetic composition, according to claim 1.

The composition according to the invention is non-colouring. According to the present invention, the term "non-colouring composition" means a composition not containing any dye for keratin fibres such as direct dyes or oxidation dye precursors (bases and/or couplers). If they are present, their content does not exceed 0.005% by weight relative to the total weight of the composition. Specifically, at such a content, only the composition would be dyed, i.e. no dyeing effect would be observed on the keratin fibres.
In the present description, the term "at least one" is equivalent to the term "one or more" and can be replaced therewith.
In the present description, the term "between" is equivalent to the term "ranging from" and can be replaced therewith; in these terms, the limits are considered as being included.

### Associative nonionic polymer

The composition according to the invention thus comprises one or more associative nonionic polymers.
For the purposes of the present invention, the term "polymer" means any compound derived from the polymerization by polycondensation or from the radical polymerization of monomers, at least one of which is other than an alkylene oxide, and of a monofunctional compound of formula RX, R denoting an optionally hydroxylated C10-C30 alkyl or alkenyl group, and X denoting a carboxylic acid, amine, amide, hydroxyl or ester group. Any compound derived solely from the simple condensation of an alkylene oxide with a fatty alcohol, a fatty ester, a fatty acid, a fatty amide or a fatty amine is in particular excluded.
For the purposes of the present invention, the term "associative polymer" means an amphiphilic polymer that is capable, in an aqueous medium, of reversibly combining with itself or with other molecules. It generally comprises in its chemical structure at least one hydrophilic zone or group and at least one hydrophobic zone or group.
The term "hydrophobic group" means a radical or polymer comprising a saturated or unsaturated and linear or branched hydrocarbon-based chain. When the hydrophobic group denotes a hydrocarbon-based radical, it comprises at least 10 carbon atoms, preferably from 10 to 30 carbon atoms, in particular from 12 to 30 carbon atoms and preferentially from 18 to 30 carbon atoms. Preferentially, the hydrocarbon-based group is derived from a monofunctional compound. By way of example, the hydrophobic group may be derived from a fatty alcohol, such as stearyl alcohol, dodecyl alcohol or decyl alcohol, or else from a polyalkylenated fatty alcohol, such as Steareth-100. It may also denote a hydrocarbon-based polymer, for instance polybutadiene.

The nonionic associative polymer(s) are chosen from polyurethane polyethers.

The associative nonionic polyurethane polyethers according to the invention comprise at least two hydrocarbon-based lipophilic chains containing from 6 to 30 carbon atoms, separated by a hydrophilic block, the hydrocarbon-based chains possibly being pendent chains or chains at the end of the hydrophilic block. In particular, it is possible for one or more pendent chains to be envisaged. In addition, the polymer may comprise a hydrocarbon-based chain at one end or at both ends of a hydrophilic block.

Preferably, the associative nonionic polyurethane polyethers according to the invention bear the hydrophobic grafts at the end of the chain (telechelic polymers).

The associative nonionic polyurethane polyethers according to the invention are triblock copolymers in which the hydrophilic block is a polyoxyethylene chain comprising from 50 to 1000 and especially from 100 to 300 oxyethylene groups; and comprising at least two hydrocarbon-based lipophilic chains containing from 6 to 30 carbon atoms, separated by the said hydrophilic block, the said hydrocarbon-based lipophilic chains possibly being pendent chains or chains at the end of a hydrophilic block.

The associative nonionic polyurethane polyethers comprise a urethane bond between the hydrophilic blocks, whence arises the name. By extension, also included among the associative nonionic polyurethane polyethers comprising a hydrophobic chain are those in which the hydrophilic blocks are linked to the hydrophobic blocks via other chemical bonds.

Preferably, the associative nonionic polyurethane polyethers according to the invention have a mass-average molecular weight (Mw) of less than or equal to 500 000 and better still less than or equal to 100 000.

Preferably, use is made of an associative nonionic polyurethane polyether that may be obtained by polycondensation of at least three compounds comprising (i) at least one polyethylene glycol comprising from 100 to 180 mol of ethylene oxide, (ii) a polyoxyethylenated stearyl alcohol comprising 100 mol of ethylene oxide, and (iii) a diisocyanate.
Such a polymer is especially sold by the company Elementis under the name Rheolate FX 1100®, which is a polycondensate of polyethylene glycol containing 136 mol of ethylene oxide, of stearyl alcohol polyoxyethylenated with 100 mol of ethylene oxide and of hexamethylene diisocyanate (HDI) with a weight-average molecular weight (Mw) of 30 000 (INCI name: PEG-136/Steareth-100/HDI Copolymer).

According to another preference, use is made of an associative nonionic polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.
Such polymers are especially sold by the company Röhm & Haas under the names Aculyn 46® and Aculyn 44®.
Aculyn 46® is a polycondensate of polyethylene glycol comprising 150 or 180 mol of ethylene oxide, of stearyl alcohol and of methylenebis(4-cyclohexyl isocyanate) (SMDI) at 15% by weight in a matrix of maltodextrin (4%) and water (81%) (INCI name: PEG-150/Stearyl Alcohol/SMDI Copolymer).
Aculyn 44® is a polycondensate of polyethylene glycol comprising 150 or 180 mol of ethylene oxide, of decyl alcohol and of methylenebis(4-cyclohexyl isocyanate) (SMDI) at 35% by weight in a mixture of propylene glycol (39%) and water (26%) (INCI name: PEG-150/Decyl Alcohol/SMDI Copolymer).

The composition according to the invention comprises associative nonionic polymers in an amount ranging from 2% to 60% by weight, preferably from 2.5% to 40% by weight, better still from 2.7% to 20% by weight or even from 2.75% to 15% by weight, relative to the total weight of the composition.

### Carboxylate anionic surfactant

The composition according to the invention also comprises one or more carboxylate anionic surfactants as defined in claim 1.

They may be chosen advantageously from acylglycinates, acylsarcosinates, acyllactylates and acylglutamates, the acyl groups preferably comprising from 14 to 30 carbon atoms and better still from 16 to 22 carbon atoms; and also the corresponding salified forms. These compounds may be optionally oxyethylenated and then preferably comprise from 1 to 50 ethylene oxide units and better still from 1 to 10 ethylene oxide units.

Mention may also be made of polyoxyalkylenated (C14-30)alkyl ether carboxylic acids, polyoxyalkylenated (C14-30)alkyl(C6-30)aryl ether carboxylic acids, polyoxyalkylenated (C14-30)alkylamido ether carboxylic acids and salts thereof, in particular those comprising from 2 to 50 ethylene oxide units, and mixtures thereof.

Salts that may be mentioned in particular include alkali metal salts such as sodium or potassium salts, ammonium salts, amine salts, amino alcohol salts or alkaline-earth metal salts, for example magnesium salts.
Amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.
Alkali metal or alkaline-earth metal salts and in particular the sodium or magnesium salts are preferably used.

Preferentially, the carboxylate anionic surfactants are chosen from (C14-C30)acylglutamates and in particular stearoylglutamates, lauroylglutamates and cocoylglutamates; (C14-C30)acylsarcosinates and in particular palmitoylsarcosinates, stearoylsarcosinates, lauroylsarcosinates and cocoylsarcosinates; (C14-C30)acyllactylates and in particular behenoyllactylates, lauroyllactylates and (iso)stearoyllactylates; alkyl ether carboxylates and in particular lauryl ether carboxylates; and mixtures thereof, in particular in the form of alkali metal, alkaline-earth metal, ammonium, amine or amino alcohol salts.
Mention may be made more particularly of disodium cocoylglutamate and sodium lauroylsarcosinate.

The composition according to the invention comprises carboxylate anionic surfactant(s) in an amount ranging from 0.1% to 10% by weight, preferably from 1% to 8% by weight and preferentially from 1.5% to 7.5% by weight, relative to the total weight of the composition.

### Additional surfactants

The composition according to the invention may also comprise one or more additional surfactants, preferably chosen from nonionic surfactants, anionic surfactants other than the above carboxylate anionic surfactants, and amphoteric surfactants.

Examples of additional anionic surfactants that may be mentioned include alkyl sulfates, alkyl ether sulfates, alkylamido ether sulfates, alkylaryl polyether sulfates, monoglyceride sulfates, alkyl sulfonates, alkylamide sulfonates, alkylaryl sulfonates, α-olefin sulfonates, paraffin sulfonates, alkyl sulfoacetates, alkylsulfosuccinates, alkyl ether sulfosuccinates, alkylamide sulfosuccinates, acylisethionates and N-acyltaurates, and also the corresponding salified forms, the alkyl and acyl groups of these compounds comprising from 6 to 24 carbon atoms, and the aryl group denoting a phenyl group; these compounds may be oxyethylenated and then preferably comprise from 1 to 50 ethylene oxide units and better still from 1 to 10 ethylene oxide units.

When the anionic surfactants are in salt form, they may be chosen from alkali metal salts such as the sodium or potassium salt and preferably the sodium salt, ammonium salts, amine salts and in particular amino alcohol salts or alkaline-earth metal salts such as the magnesium salts. Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts. Alkali metal or alkaline-earth metal salts, and in particular sodium or magnesium salts, are preferably used.

Among the additional anionic surfactants, (C6-C24)acylisethionates, (C6-C24)alkyl sulfosuccinates and (C6-C24)alkyl sulfates, and also (C6-C24)alkyl ether sulfosuccinates and (C6-C24)alkyl ether sulfates comprising from 2 to 50 ethylene oxide units are most particularly preferred; these compounds may be in the form of alkali metal, ammonium, amino alcohol or alkaline-earth metal salts, or a mixture of these compounds.
Better still, (C12-C20)acylisethionates, (C12-C20)alkyl sulfates and (C12-C20)alkyl ether sulfates comprising from 2 to 20 ethylene oxide units, especially in the form of alkali metal, ammonium, amino alcohol and alkaline-earth metal salts, or a mixture of these compounds, are preferred.

The additional nonionic surfactants that may be used may be chosen from alcohols, α-diols and (C₁₋₂₀)alkylphenols, these compounds being polyethoxylated, polypropoxylated or bearing a fatty chain comprising, for example, from 8 to 30 carbon atoms and especially from 16 to 30 carbon atoms, the number of ethylene oxide and/or propylene oxide groups possibly ranging especially from 2 to 50, and the number of glycerol groups possibly ranging especially from 2 to 30.
Mention may also be made of condensates of ethylene oxide and of propylene oxide with fatty alcohols; polyethoxylated fatty amides preferably having from 2 to 30 ethylene oxide units, polyglycerolated fatty amides containing on average from 1 to 5, and in particular from 1.5 to 4, glycerol groups; ethoxylated fatty acid esters of sorbitan preferably containing from 2 to 40 ethylene oxide units, fatty acid esters of sucrose, fatty acid esters of polyethylene glycol, N-(C6-24 alkyl)glucamine derivatives, amine oxides such as (C10-14alkyl)amine oxides or N-(C10-14 acyl)aminopropylmorpholine oxides.
Preferentially, use is made of ethoxylated fatty acid esters of sorbitan and polyethoxylated fatty alcohols, and mixtures thereof.

Mention may also be made of nonionic surfactants of alkylpolyglycoside type, represented especially by the following general formula: R₁O-(R₂O)ₜ-(G)ᵥ
in which:
- R₁ represents a linear or branched alkyl or alkenyl radical comprising 6 to 24 carbon atoms and especially 8 to 18 carbon atoms, or an alkylphenyl radical whose linear or branched alkyl radical comprises from 6 to 24 carbon atoms and especially 8 to 18 carbon atoms,
- R₂ represents an alkylene radical comprising 2 to 4 carbon atoms,
- G represents a sugar unit comprising 5 to 6 carbon atoms,
- t denotes a value ranging from 0 to 10 and preferably 0 to 4,
- v denotes a value ranging from 1 to 15 and preferably 1 to 4.
Preferably, the alkylpolyglycoside surfactants are compounds having the formula described above in which R₁ denotes a saturated or unsaturated, linear or branched alkyl radical comprising from 8 to 18 carbon atoms, t denotes a value ranging from 0 to 3, preferably equal to 0, G denotes glucose, fructose or galactose, preferably glucose; the degree of polymerization, i.e. the value of v, may range from 1 to 15 and preferably from 1 to 4; the mean degree of polymerization more particularly being between 1 and 2.
The glucoside bonds between the sugar units are generally of 1-6 or 1-4 type and preferably of 1-4 type. Preferably, the alkylpolyglycoside surfactant is an alkylpoly-glucoside surfactant.
Among the commercial products, mention may be made of the products sold by the company Cognis under the names Plantaren® (600 CS/U, 1200 and 2000) or Plantacare® (818, 1200 and 2000); the products sold by the company SEPPIC under the names Triton CG110 (or Oramix® CG 10) and Triton CG312 (or Oramix NS 10); the products sold by the company BASF under the name Lutensol GD 70, or the products sold by the company Chem Y under the name AG10 LK. Preferably, use is made of C8/C16-alkyl polyglycosides 1,4, especially as an aqueous 53% solution, such as the product sold by Cognis under the reference Plantacare® 818 UP.

Preferably, the additional nonionic surfactant(s) are chosen from surfactants of alkylpolyglycoside type.

The additional amphoteric surfactants that may be used in the invention may be optionally quaternized secondary or tertiary aliphatic amine derivatives, in which the aliphatic group is a linear or branched chain comprising from 8 to 22 carbon atoms, said amine derivatives containing at least one anionic group, for instance a carboxylate, sulfonate, sulfate, phosphate or phosphonate group.
Mention may be made in particular of (C8-C20)alkylbetaines, sulfobetaines, alkyl(C8-C20)sulfobetaines, (C8-C20)alkylamido(C1-C6)alkylbetaines, such as co-camidopropylbetaine, and (C8-C20)alkylamido(C1-C6)alkylsulfobetaines.

Among the optionally quaternized secondary or tertiary aliphatic amine derivatives that may be used, mention may also be made of the products having the following respective structures (A2) and (A3):

(A2) Rₐ-CON(Z)CH₂-(CH₂)ₘ-N⁺(R_{b})(R_{c})(CH₂COO⁻)

in which:
Rₐ represents a C₁₀-C₃₀ alkyl or alkenyl group derived from an acid Rₐ-COOH preferably present in hydrolysed coconut oil, or a heptyl, nonyl or undecyl group;
R_{b} represents a β-hydroxyethyl group;
R_{c} represents a carboxymethyl group;
m is equal to 0, 1 or 2;
Z represents a hydrogen atom or a hydroxyethyl or carboxymethyl group;

   (A3) R_{a'}-CON(Z)CH₂₋(CH₂)_{m'}-N(B)(B')

   in which:
   B represents -CH₂CH₂OX', with X' representing -CH₂-COOH, CH₂-COOZ',-CH₂CH₂-COOH, -CH₂CH₂-COOZ', or a hydrogen atom,
   B' represents -(CH₂)_{z}-Y', with z = 1 or 2, and Y' representing -COOH, -COOZ',-CH₂-CHOH-SO₃H or -CH₂-CHOH-SO₃Z',
   m' is equal to 0, 1 or 2,
   Z represents a hydrogen atom or a hydroxyethyl or carboxymethyl group;
   Z' represents an ion resulting from an alkali metal or alkaline-earth metal, such as sodium, potassium or magnesium; an ammonium ion; or an ion resulting from an organic amine and in particular from an amino alcohol, such as monoethanolamine, diethanolamine and triethanolamine, monoisopropanolamine, diisopropanolamine or triisopropanolamine, 2-amino-2-methyl-1-propanol, 2-amino-2-methyl-1,3-propanediol and tris(hydroxymethyl)aminomethane,
   R_{a'} represents a C₁₀-C₃₀ alkyl or alkenyl group of an acid R_{a'}COOH preferably present in hydrolysed linseed oil or coconut oil, an alkyl group, in particular a C₁₇ alkyl group, and its iso form, or an unsaturated C₁₇ group.

The compounds corresponding to formula (A3) are preferred. These compounds are also classified in the CTFA dictionary, 5th edition, 1993, under the names disodium cocoamphodiacetate, disodium lauroamphodiacetate, disodium caprylamphodiacetate, disodium caprylamphodiacetate, disodium cocoamphodipropionate, disodium lauroamphodipropionate, disodium caprylamphodipropionate, disodium caprylamphodipropionate, lauroamphodipropionic acid, cocoamphodipropionic acid.
Examples that may be mentioned include the cocoamphodiacetate sold by the company Rhodia under the trade name Miranol® C2M Concentrate or under the trade name Miranol Ultra C 32 and the product sold by the company Chimex under the trade name Chimexane HA.

Use may also be made of compounds of formula (A4):

(A4) R_{a"}-NH-CH(Y")-(CH₂)n-C(O)-NH-(CH₂)n'-N(R_{d})(Rₑ)

in which:
- R_{a"} represents a C₁₀-C₃₀ alkyl or alkenyl group of an acid
   R_{a"}-C(O)OH, which is preferably present in hydrolysed linseed oil or coconut oil;
- Y" represents the group -C(O)OH, -C(O)OZ", -CH₂-CH(OH)-SO₃H or the group-CH₂-CH(OH)-SO₃-Z", with Z" representing a cationic counterion resulting from an alkali metal or alkaline-earth metal, such as sodium, an ammonium ion or an ion resulting from an organic amine;
- R_{d} and Rₑ represent, independently of each other, a C1-C4 alkyl or hydroxyalkyl radical; and
- n and n' denote, independently of each other, an integer ranging from 1 to 3. Mention may in particular be made of the compound classified in the CTFA dictionary under the name sodium diethylaminopropyl cocoaspartamide and sold by the company Chimex under the name Chimexane HB.

Preferably, the amphoteric surfactants are chosen from (C8-C20)alkyl betaines, (C8-C20)alkylamido(C1-C6)alkyl betaines and (C8-C20)alkylamphodiacetates, and also the sodium salt of diethylaminopropyl laurylaminosuccinamate, and mixtures thereof.

The composition according to the invention preferably comprises a total amount of nonionic surfactants (optional), anionic surfactants (total, i.e. carboxylates and optional additional) and amphoteric surfactants (optional) ranging from 0.1% to 10% by weight, preferably ranging from 1% to 8% by weight and preferentially from 1.5% to 7.5% by weight, relative to the total weight of the composition.
The composition according to the invention preferably comprises a total amount of surfactants (cationic, anionic, nonionic, amphoteric and zwitterionic) ranging from 0.1% to 10% by weight, preferably ranging from 1% to 8% by weight and preferentially from 1.5% to 7.5% by weight, relative to the total weight of the composition.

Preferably, the ratio (weight percentage) "nonionic surfactants (optional) + anionic surfactants (total) + amphoteric surfactants (optional)"/"nonionic associative polymers" is less than or equal to 3; it preferably ranges from 0.01 to 3, especially from 0.01 to 2.8 and preferentially from 0.1 to 2.5.

### Polymers

The composition according to the invention may also comprise one or more polymers other than the nonionic associative polymers according to the invention, especially chosen from amphoteric and cationic polymers, and also mixtures thereof.

The term "cationic polymer" means any polymer comprising cationic groups and/or groups that can be ionized to cationic groups. Preferably, the cationic polymer is hydrophilic or amphiphilic. The preferred cationic polymers are chosen from those that contain units comprising primary, secondary, tertiary and/or quaternary amine groups that may either form part of the main polymer chain or may be borne by a side substituent directly connected thereto.
The cationic polymers that may be used preferably have a weight-average molar mass (Mw) of between 500 and 5×10⁶ approximately and preferably between 10³ and 3×10⁶ approximately.

Among the cationic polymers, mention may be made more particularly of:
(1) homopolymers or copolymers derived from acrylic or methacrylic esters or amides and comprising at least one of the units of the following formulae: 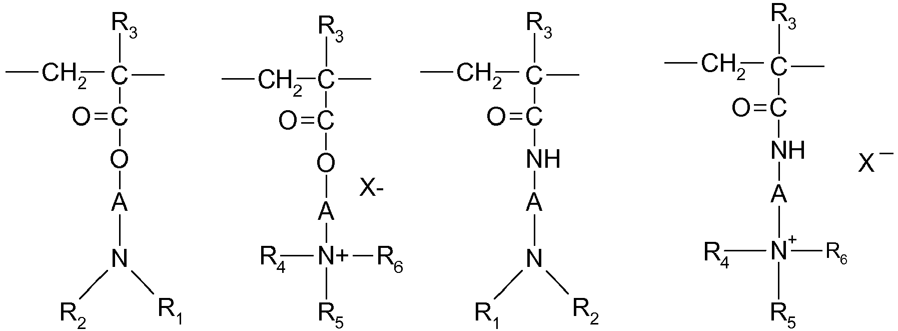 in which:
   - R3, which may be identical or different, denote a hydrogen atom or a CH₃ radical;
   - A, which may be identical or different, represent a linear or branched divalent alkyl group of 1 to 6 carbon atoms, preferably 2 or 3 carbon atoms, or a hydroxyalkyl group of 1 to 4 carbon atoms;
   - R4, R5 and R6, which may be identical or different, represent an alkyl group containing from 1 to 18 carbon atoms or a benzyl radical, preferably an alkyl group containing from 1 to 6 carbon atoms;
   - R1 and R2, which may be identical or different, represent a hydrogen atom or an alkyl group containing from 1 to 6 carbon atoms, preferably methyl or ethyl;
   - X denotes an anion derived from a mineral or organic acid, such as a methosulfate anion or a halide such as chloride or bromide.

   The copolymers of family (1) may also contain one or more units derived from comonomers that may be selected from the family of acrylamides, methacrylamides, diacetone acrylamides, acrylamides and methacrylamides substituted on the nitrogen with lower (C₁-C₄) alkyls, acrylic or methacrylic acids or esters thereof, vinyllactams such as vinylpyrrolidone or vinylcaprolactam, and vinyl esters.
   Among these copolymers of family (1), mention may be made of:
   - copolymers of acrylamide and of dimethylaminoethyl methacrylate quaternized with dimethyl sulfate or with a dimethyl halide, such as the product sold under the name Hercofloc by the company Hercules,
   - copolymers of acrylamide and of methacryloyloxyethyltrimethylammonium chloride, such as those sold under the name Bina Quat P 100 by the company Ciba Geigy,
   - the copolymer of acrylamide and of methacryloyloxyethyltrimethylammonium methosulfate, such as the product sold under the name Reten by the company Hercules,
   - quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in detail in French patents 2 077 143 and 2 393 573,
   - dimethylaminoethyl methacrylate/vinylcaprolactam/vinylpyrrolidone terpolymers, such as the product sold under the name Gaffix VC 713 by the company ISP,
   - vinylpyrrolidone/methacrylamidopropyldimethylamine copolymers, such as those sold in particular under the name Styleze CC 10 by ISP,
   - quaternized vinylpyrrolidone/dimethylaminopropylmethacrylamide copolymers such as the product sold under the name Gafquat HS 100 by the company ISP,
   - preferably crosslinked polymers of methacryloyloxy(C₁-C₄)alkyltri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with an olefinically unsaturated compound, more particularly methylenebisacrylamide. A crosslinked acrylamide/methacryloyloxyethyltrimethylammonium chloride copolymer (20/80 by weight) in the form of a dispersion containing 50% by weight of said copolymer in mineral oil may be used more particularly. This dispersion is sold under the name Salcare® SC 92 by the company Ciba. A crosslinked methacryloyloxyethyltrimethylammonium chloride homopolymer containing about 50% by weight of the homopolymer in mineral oil or in a liquid ester can also be used. These dispersions are sold under the names Salcare® SC 95 and Salcare® SC 96 by the company Ciba.
(2) Cationic polysaccharides, especially cationic celluloses and galactomannan gums. Among the cationic polysaccharides, mention may be made more particularly of cellulose ether derivatives comprising quaternary ammonium groups, cationic cellulose copolymers or cellulose derivatives grafted with a water-soluble quaternary ammonium monomer and cationic galactomannan gums.
   The cellulose ether derivatives comprising quaternary ammonium groups are especially described in French patent 1 492 597, and mention may be made of the polymers sold under the name Ucare Polymer "JR" (JR 400 LT, JR 125 and JR 30M) or "LR" (LR 400 or LR 30M) by the company Amerchol. These polymers are also defined in the CTFA dictionary as quaternary ammoniums of hydroxyethyl cellulose that have reacted with an epoxide substituted with a trimethylammonium group.
   Cationic cellulose copolymers or cellulose derivatives grafted with a water-soluble quaternary ammonium monomer are described especially in US patent 4 131 576, and mention may be made of hydroxyalkyl celluloses, for instance hydroxymethyl-, hydroxyethyl- or hydroxypropylcelluloses grafted, in particular, with a methacryloylethyltrimethylammonium, methacrylamidopropyltrimethylammonium or dimethyldiallylammonium salt. The commercial products corresponding to this definition are more particularly the products sold under the names Celquat L 200 and Celquat H 100 by the company National Starch.
   The cationic galactomannan gums are described more particularly in US patents 3 589 578 and 4 031 307, and mention may be made of guar gums comprising cationic trialkylammonium groups. Use is made, for example, of guar gums modified with a 2,3-epoxypropyltrimethylammonium salt (for example, the chloride). Such products are especially sold under the trade names Jaguar C13 S, Jaguar C 15, Jaguar C 17 or Jaguar C162 by the company Rhodia.
(3) Polymers formed from piperazinyl units and divalent alkylene or hydroxyalkylene radicals containing straight or branched chains, optionally interrupted with oxygen, sulfur or nitrogen atoms or with aromatic or heterocyclic rings, and also the oxidation and/or quaternization products of these polymers.
(4) Water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they comprise one or more tertiary amine functions, they can be quaternized.
(5) Polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical comprises from 1 to 4 carbon atoms and preferably denotes methyl, ethyl or propyl. Among these derivatives, mention may be made more particularly of the adipic acid/dimethylaminohydroxypropyl/diethylenetriamine polymers sold under the name Cartaretine F, F4 or F8 by the company Sandoz.
(6) Polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyamino amide being reacted with epichlorohydrin in a mole ratio of epichlorohydrin relative to the secondary amine group of the polyamino amide preferably of between 0.5:1 and 1.8:1. Polymers of this type are sold in particular under the name Hercosett 57 by the company Hercules Inc. or alternatively under the name PD 170 or Delsette 101 by the company Hercules in the case of the adipic acid/epoxypropyl/diethylenetriamine copolymer.
(7) Cyclopolymers of alkyldiallylamine or of dialkyldiallylammonium, such as the homopolymers or copolymers containing, as main constituent of the chain, units corresponding to formula (I) or (II): 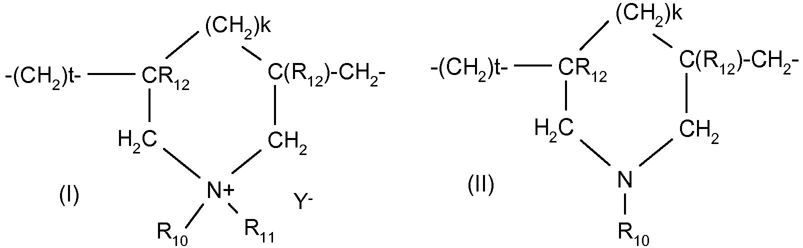 in which:
   - k and t are equal to 0 or 1, the sum k + t being equal to 1;
   - R12 denotes a hydrogen atom or a methyl radical;
   - R10 and R11, independently of each other, denote an alkyl group containing from 1 to 6 carbon atoms, a hydroxyalkyl group in which the alkyl group contains 1 to 5 carbon atoms, a C1-C4 amidoalkyl group; or alternatively R10 and R11 may denote, together with the nitrogen atom to which they are attached, heterocyclic groups such as piperidyl or morpholinyl; R10 and R11, independently of each other, preferably denote an alkyl group containing from 1 to 4 carbon atoms;
   - Y⁻ is an anion such as bromide, chloride, acetate, borate, citrate, tartrate, bisulfate, bisulfite, sulfate or phosphate.

   Mention may be made more particularly of the dimethyldiallylammonium salt (for example chloride) homopolymer sold under the name Merquat 100 by the company Nalco (and homologues thereof of low weight-average molar masses) and the copolymers of diallyldimethylammonium salts (for example chloride) and of acrylamide, sold especially under the names Merquat 550 and Merquat 7SPR.
(8) quaternary diammonium polymers comprising repeating units of formula: 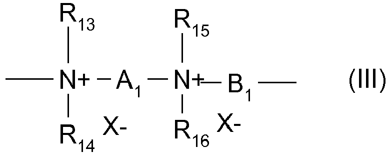 in which:
   - R13, R14, R15 and R16, which may be identical or different, represent aliphatic, alicyclic or arylaliphatic radicals comprising from 1 to 20 carbon atoms, or lower hydroxyalkylaliphatic radicals, or else R13, R14, R15 and R16, together or separately, constitute, with the nitrogen atoms to which they are attached, heterocycles optionally comprising a second non-nitrogen heteroatom, or else R13, R14, R15 and R16 represent a linear or branched C₁-C₆ alkyl radical substituted with a nitrile, ester, acyl, amide or -CO-O-R17-D or -CO-NH-R17-D group in which R17 is an alkylene and D is a quaternary ammonium group;
   - A1 and B1 represent divalent polymethylene groups comprising from 2 to 20 carbon atoms, which may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
   - X⁻ denotes an anion derived from a mineral or organic acid;
      it being understood that A1, R13 and R15 can form, with the two nitrogen atoms to which they are attached, a piperazine ring;
      in addition, if A1 denotes a linear or branched, saturated or unsaturated alkylene or hydroxyalkylene radical, B1 may also denote a group (CH₂)ₙ-CO-D-OC-(CH₂)ₙ- in which D denotes:
      a) a glycol residue of formula -O-Z-O-, in which Z denotes a linear or branched hydrocarbon-based radical, or a group corresponding to one of the following formulae: -(CH₂-CH₂-O)ₓ-CH₂-CH₂-; -[CH₂-CH(CH₃)-O]_{y}-CH₂-CH(CH₃)-, where x and y denote an integer from 1 to 4, representing a defined and unique degree of polymerization or any number from 1 to 4 representing an average degree of polymerization;
      b) a bis-secondary diamine residue such as a piperazine derivative;
      c) a bis-primary diamine residue of formula: -NH-Y-NH-, where Y denotes a linear or branched hydrocarbon-based radical, or else the divalent radical -CH₂-CH₂-S-S-CH₂-CH₂-;
      d) a ureylene group of formula: -NH-CO-NH-.

   Preferably, X⁻ is an anion such as chloride or bromide. These polymers have a number-average molar mass (Mn) generally of between 1000 and 100 000.
   Mention may be made more particularly of polymers that are composed of repeating units corresponding to the formula: 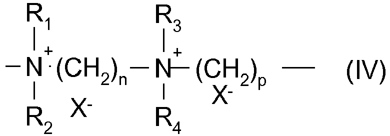 in which R1, R2, R3 and R4, which may be identical or different, denote an alkyl or hydroxyalkyl radical containing from 1 to 4 carbon atoms approximately, n and p are integers ranging from 2 to 20 approximately, and X⁻ is an anion derived from a mineral or organic acid.
   A particularly preferred compound of formula (IV) is that for which R1, R2, R3 and R4 represent a methyl radical and n = 3, p = 6 and X = Cl, known as Hexadime-thrine chloride according to the INCI (CTFA) nomenclature.
(9) Polyquaternary ammonium polymers comprising units of formula (V): 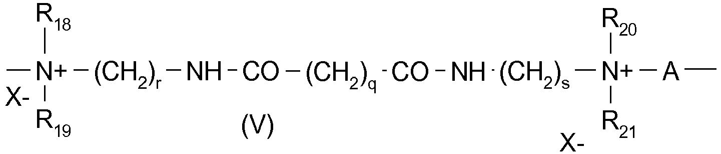 in which:
   - R18, R19, R20 and R21, which may be identical or different, represent a hydrogen atom or a methyl, ethyl, propyl, β-hydroxyethyl, β-hydroxypropyl or CH₂CH₂(OCH₂CH₂)ₚOH radical, in which p is equal to 0 to or an integer between 1 and 6, with the proviso that R18, R19, R20 and R21 do not simultaneously represent a hydrogen atom,
   - r and s, which may be identical or different, are integers between 1 and 6,
   - q is equal to 0 or to an integer between 1 and 34,
   - X- denotes an anion such as a halide,
   - A denotes a dihalide radical or preferably represents -CH₂-CH₂-O-CH₂-CH₂-. Examples that may be mentioned include the products Mirapol® A 15, Mirapol® AD1, Mirapol® AZ1 and Mirapol® 175 sold by the company Miranol.
(10) Quaternary polymers of vinylpyrrolidone and of vinylimidazole, for instance the products sold under the names Luviquat® FC 905, FC 550 and FC 370 by the company BASF.
(11) Polyamines such as Polyquart® H sold by Cognis, referred to under the name Polyethylene glycol (15) tallow polyamine in the CTFA dictionary.
(12) Polymers comprising in their structure:
   (b) one or more units corresponding to formula (A) below: 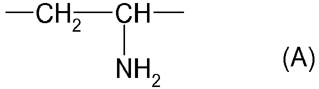
   (b) optionally, one or more units corresponding to formula (B) below:

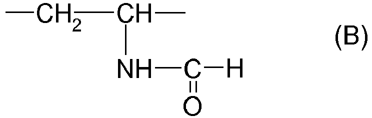

In other words, these polymers may be chosen especially from homopolymers or copolymers comprising one or more units derived from vinylamine and optionally one or more units derived from vinylformamide.
Preferably, these cationic polymers are chosen from polymers comprising, in their structure, from 5 mol% to 100 mol% of units corresponding to formula (A) and from 0 to 95 mol% of units corresponding to formula (B), preferentially from 10 mol% to 100 mol% of units corresponding to formula (A) and from 0 to 90 mol% of units corresponding to formula (B).
These polymers may be obtained, for example, by partial hydrolysis of polyvinyl-formamide. This hydrolysis may be performed in an acidic or basic medium.
The weight-average molecular mass of the said polymer, measured by light scattering, may range from 1000 to 3 000 000 g/mol, preferably from 10 000 to 1 000 000 g/mol and more particularly from 100 000 to 500 000 g/mol.
The cationic charge density of these polymers may range from 2 meq/g to 20 meq/g, preferably from 2.5 to 15 meq/g and more particularly from 3.5 to 10 meq/g.
The polymers comprising units of formula (A) and optionally units of formula (B) are sold especially under the name Lupamin by the company BASF, for instance, in a non-limiting manner, the products sold under the names Lupamin 9095, Lupamin 5095, Lupamin 1095, Lupamin 9030 (or Luviquat 9030) and Lupamin 9010.

Other cationic polymers that may be used in the context of the invention are cationic proteins or cationic protein hydrolyzates, polyalkyleneimines, in particular polyethyleneimines, polymers comprising vinylpyridine or vinylpyridinium units, condensates of polyamines and of epichlorohydrin, quaternary polyureylenes and chitin derivatives.

Preferably, the cationic polymers are chosen from the polymers of families (1), (2), (7) and (10) mentioned above.
Among the cationic polymers mentioned above, the ones that may preferably be used are cationic polysaccharides, especially cationic celluloses and galactomannan gums, and in particular quaternary cellulose ether derivatives such as the products sold under the name JR 400 by the company Amerchol, cationic cyclopolymers, in particular dimethyldiallylammonium salt (for example chloride) homopolymers or copolymers, products sold under the names Merquat 100, Merquat 550 and Merquat S by the company Nalco, and homologues thereof of low weight-average molecular weights, quaternary polymers of vinylpyrrolidone and of vinylimidazole, optionally crosslinked homopolymers or copolymers of methacryloyloxy(C₁-C₄)alkyltri(C₁-C₄)alkylammonium salts, and mixtures thereof.

It is also possible to use amphoteric polymers, which may preferably be chosen from amphoteric polymers comprising a repetition of:
(i) one or more units derived from a monomer of (meth)acrylamide type,
(ii) one or more units derived from a monomer of (meth)acrylamidoalkyltrialkylammonium type, and
(iii) one or more units derived from an acidic monomer of (meth)acrylic acid type.

Preferably, the units derived from a monomer of (meth)acrylamide type (i) are units of structure (Ia) below: 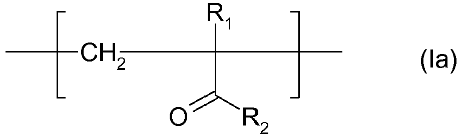 in which R₁ denotes H or CH₃ and R₂ is chosen from an amino, dimethylamino, tert-butylamino, dodecylamino or -NH-CH₂OH radical.
Preferably, the said amphoteric polymer comprises a repetition of only one unit of formula (Ia).
The unit derived from a monomer of (meth)acrylamide type of formula (Ia) in which R₁ denotes H and R₂ is an amino radical (NH₂) is particularly preferred. It corresponds to the acrylamide monomer per se.

Preferably, the units derived from a monomer of (meth)acrylamidoalkyltrialkylammonium type (ii) are units of structure (IIa): 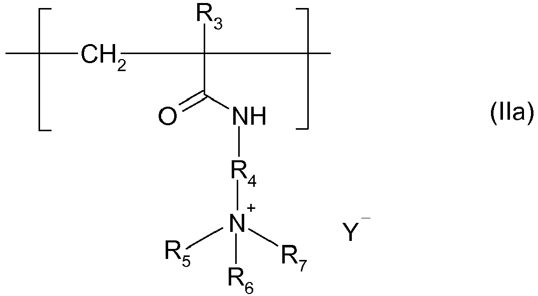 in which:
- R₃ denotes H or CH₃,
- R₄ denotes a group (CH₂)ₖ with k being an integer ranging from 1 to 6 and preferably from 2 to 4;
- R₅, R₆, and R₇, which may be identical or different, each denote an alkyl group containing from 1 to 4 carbon atoms;
- Y⁻ is an anion such as bromide, chloride, acetate, borate, citrate, tartrate, bisulfate, bisulfite, sulfate or phosphate.
Preferably, the said amphoteric polymer comprises a repetition of only one unit of formula (IIa).
Among these units derived from a monomer of (meth)acrylamidoalkyltrialkylammonium type of formula (IIa), the ones that are preferred are those derived from the methacrylamidopropyltrimethylammonium chloride monomer, for which R₃ denotes a methyl radical, k is equal to 3, R₅, R₆ and R₇ denote a methyl radical, and Y⁻ denotes a chloride anion.

Preferably, the units derived from a monomer of (meth)acrylic acid type (iii) are units of formula (IIIa): 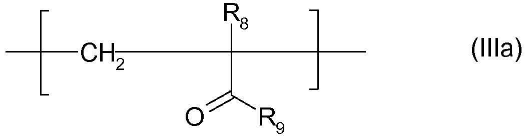 in which R₈ denotes H or CH₃ and R₉ denotes a hydroxyl radical or a -NH-C(CH₃)₂-CH₂-SO₃H radical.
The preferred units of formula (IIIa) correspond to the acrylic acid, methacrylic acid and 2-acrylamino-2-methylpropanesulfonic acid monomers.
Preferably, the unit derived from a monomer of (meth)acrylic acid type of formula (IIIa) is that derived from acrylic acid, for which R₈ denotes a hydrogen atom and R₉ denotes a hydroxyl radical.
The acidic monomer(s) of (meth)acrylic acid type may be non-neutralized or partially or totally neutralized with an organic or mineral base.
Preferably, the said amphoteric polymer comprises a repetition of only one unit of formula (IIIa).

According to a preferred embodiment of the invention, the amphoteric polymer(s) of this type comprise at least 30 mol% of units derived from a monomer of (meth)acrylamide type (i). Preferably, they comprise from 30 mol% to 70 mol% and more preferably from 40 mol% to 60 mol% of units derived from a monomer of (meth)acrylamide type.
The content of units derived from a monomer of (meth)acrylamidoalkyltrialkylammonium type (ii) may advantageously be from 10 mol% to 60 mol% and preferentially from 20 mol% to 55 mol%.
The content of units derived from an acidic monomer of (meth)acrylic acid type (iii) may advantageously be from 1 mol% to 20 mol% and preferentially from 5mol% to 15 mol%.
According to a particularly preferred embodiment of the invention, the amphoteric polymer of this type comprises:
- from 30 mol% to 70 mol% and more preferably from 40 mol% to 60 mol% of units derived from a monomer of (meth)acrylamide type (i),
- from 10 mol% to 60 mol%, preferentially from 20 mol% to 55 mol% of units derived from a monomer of (meth)acrylamidoalkyltrialkylammonium type (ii), and
- from 1 mol% to 20 mol% and preferentially from 5 mol% to 15 mol% of units derived from a monomer of (meth)acrylic acid type (iii).

Amphoteric polymers of this type may also comprise additional units, other than the units derived from a monomer of (meth)acrylamide type, of (meth)acrylamidoalkyltrialkylammonium type and of (meth)acrylic acid type as described above.
However, according to a preferred embodiment of the invention, the said amphoteric polymers consist solely of units derived from monomers (i) of (meth)acrylamide type, (ii) of (meth)acrylamidoalkyltrialkylammonium type and (iii) of (meth)acrylic acid type.
As an example of amphoteric polymers that are particularly preferred, mention may be made of acrylamide/methylacrylamidopropyltrimethylammonium chloride/acrylic acid terpolymers. Such polymers are listed in the CTFA dictionary International Cosmetic Ingredient Dictionary, 10th edition 2004, under the name Polyquaternium 53. Corresponding products are especially sold as Merquat 2003 and Merquat 2003 PR by Nalco.

As another type of amphoteric polymer that may be used, mention may also be made of copolymers based on (meth) acrylic acid and on a dialkyldiallylammonium salt, such as copolymers of (meth) acrylic acid and of dimethyldiallylammonium chloride. An example that may be mentioned is Merquat 280 sold by the company Nalco.

The composition according to the invention may comprise the cationic and/or amphoteric polymers in an amount of between 0.01% and 5% by weight, especially from 0.05% to 3% by weight and preferentially from 0.1% to 2% by weight, relative to the total weight of the composition.

### Other ingredients

The cosmetic composition according to the invention may be in any galenical form conventionally used and especially in the form of an aqueous, alcoholic or aqueous-alcoholic or oily solution or suspension; a solution or dispersion of the lotion or serum type; an emulsion, an aqueous or anhydrous gel, or of any other cosmetic form.

The composition according to the invention is preferably aqueous and then comprises water at a concentration preferably ranging from 5% to 98% by weight, especially from 20% to 95% by weight and better still from 50% to 90% by weight, relative to the total weight of the composition.
The composition may also comprise one or more organic solvents that are liquid at 25°C and 1 atm., and especially water-soluble, such as C1-C7 alcohols, especially C1-C7 aliphatic or aromatic monoalcohols, and C3-C7 polyols and polyol ethers, which may thus be used alone or as a mixture with water. Advantageously, the organic solvent may be chosen from ethanol and isopropanol, and mixtures thereof.

The composition according to the invention may also comprise at least one common cosmetic ingredient, other than the compounds of the invention, chosen especially from plant, mineral, animal or synthetic oils; solid fatty substances and especially waxes, C8-C40 esters and C8-C40 acids; C8-C40 alcohols; cationic surfactants, anionic polymers; sunscreens; moisturizers; antidandruff agents; antioxidants; chelating agents; nacreous agents and opacifiers; plasticizers or coalescers; hydroxy acids; fillers; silicones and in particular polydimethylsiloxanes (PDMS); fragrances; basifying or acidifying agents; aldehydes, DHA; thickeners other than the nonionic associative polymers according to the invention, and preferably chosen from non-polymeric thickeners and non-associative polymeric thickeners. The composition can, of course, comprise several cosmetic ingredients appearing in the above list. Those skilled in the art will take care to choose the ingredients included in the composition and the amounts thereof so that they do not harm the properties of the compositions of the present invention.

The pH of the composition, if it is aqueous, is preferably between 3 and 9 and especially between 3 and 6.

The cosmetic composition according to the invention especially finds a particularly advantageous application in the field of haircare and hair hygiene, especially for caring for and/or cleansing the hair and/or the scalp.
The cosmetic composition may be rinsed out or left in after having been applied to the hair and/or the scalp; it is preferably rinsed out, after an optional leave-on time. The composition according to the invention may be conditioned in a tube, in a bottle optionally equipped with a pump, or alternatively in an aerosol. In the case of an aerosol, the composition may then contain one or more standard propellants.

A subject of the invention is also a cosmetic treatment process, especially for caring for and/or cleansing the hair and/or the scalp, comprising the application to the hair and/or the scalp of a cosmetic composition according to the invention, optionally followed by rinsing, after an optional leave-on time.

The invention is illustrated in greater detail in the examples that follow.

### Examples 1 to 12

The following hair compositions are prepared (weight percentage of commercial material):

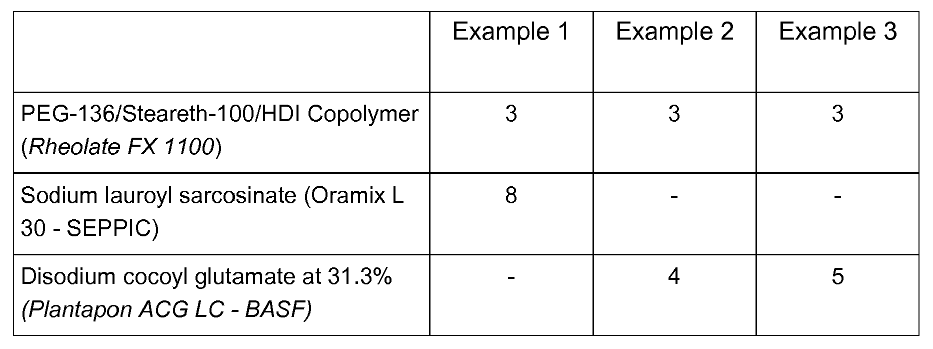

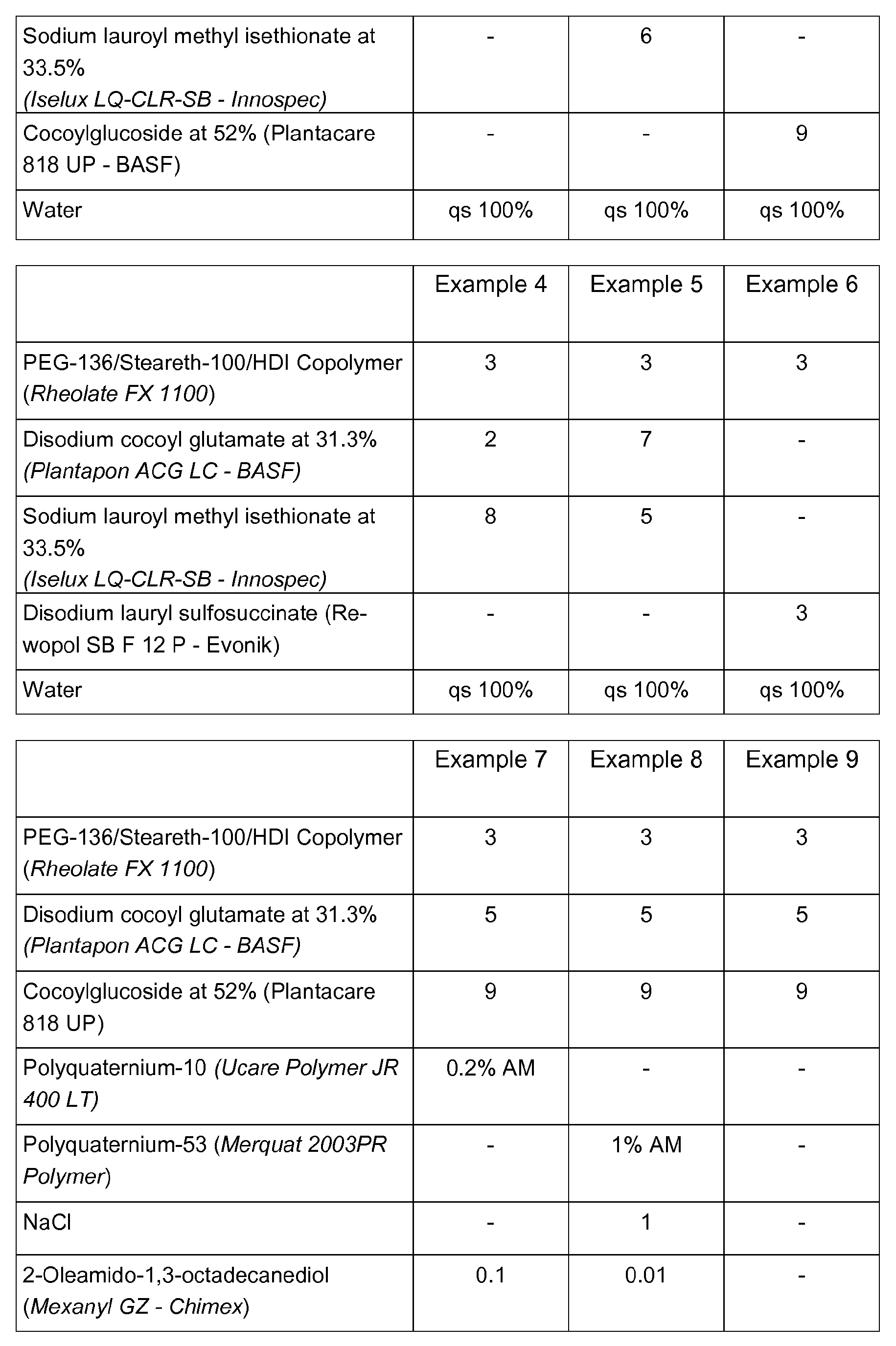

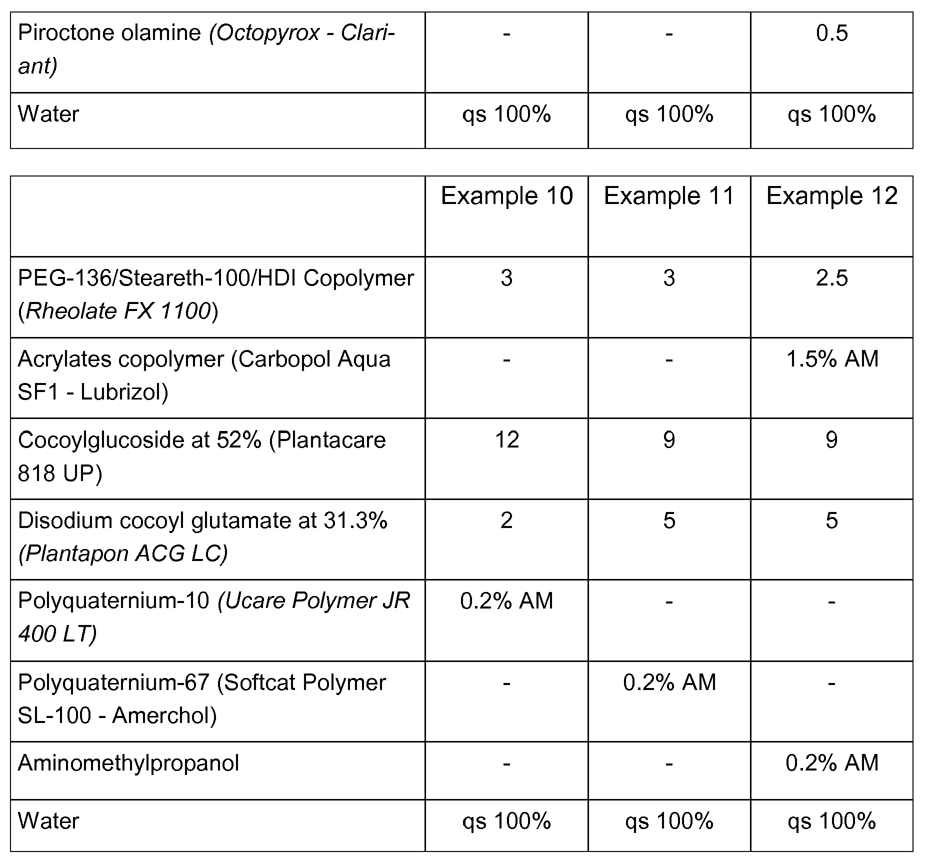

Detergent compositions with a foaming effect are obtained, which may be used for cleansing the hair, and which lead to good cosmetic properties on dry hair.

## Claims

1. Non-colouring cosmetic composition comprising:
- one or more associative nonionic polymers that are triblock polyurethane polyether copolymers in which the hydrophilic block is a polyoxyethylene chain comprising from 50 to 1000 and especially from 100 to 300 oxyethylene groups; and comprising at least two hydrocarbon-based lipophilic chains containing from 6 to 30 carbon atoms, separated by the said hydrophilic block, the said hydrocarbon-based lipophilic chains possibly being pendent chains or chains at the end of a hydrophilic block, and
- one or more carboxylate anionic surfactants, chosen from
- acylglycinates, acylsarcosinates, acyllactylates and acylglutamates, the acyl groups preferably comprising from 14 to 30 carbon atoms and better still from 16 to 22 carbon atoms; and also the corresponding salified forms; these compounds optionally being oxyethylenated and then preferably comprising from 1 to 50 ethylene oxide units and better still from 1 to 10 ethylene oxide units;
- polyoxyalkylenated (C14-30)alkyl ether carboxylic acids, polyoxyalkylenated (C14-30)alkyl(C6-30)aryl ether carboxylic acids, polyoxyalkylenated (C14-30)alkylamido ether carboxylic acids and salts thereof, in particular those comprising from 2 to 50 ethylene oxide units;
the composition comprising associative nonionic polymer(s) in an amount ranging from 2% to 60% by weight, relative to the total weight of the composition, and
the composition comprising carboxylate anionic surfactant(s) in an amount ranging from 0.1% to 10% by weight, relative to the total weight of the composition.

2. Composition according to Claim 1, in which the associative nonionic polymers are chosen from:
- associative nonionic polyurethane polyethers that may be obtained by polycondensation of at least three compounds comprising (i) at least one polyethylene glycol comprising from 100 to 180 mol of ethylene oxide, (ii) a polyoxyethylenated stearyl alcohol comprising 100 mol of ethylene oxide, and (iii) a diisocyanate;
- associative nonionic polyurethane polyethers that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

3. Composition according to any one of the preceding claims, comprising the associative nonionic polymers in an amount ranging from 2.5% to 40% by weight, better still from 2.7% to 20% by weight or even from 2.75% to 15% by weight, relative to the total weight of the composition.

4. Composition according to any one of the preceding claims, in which the carboxylate anionic surfactants are chosen from (C14-C30)acylglutamates and in particular stearoylglutamates, lauroylglutamates and cocoylglutamates; (C14-C30)acylsarcosinates and in particular palmitoylsarcosinates, stearoylsarcosinates, lauroylsarcosinates and cocoylsarcosinates; (C14-C30)acyllactylates and in particular behenoyllactylates, lauroyllactylates and (iso)stearoyllactylates; alkyl ether carboxylates and in particular lauryl ether carboxylates; and mixtures thereof, in particular in the form of alkali metal, alkaline-earth metal, ammonium, amine or amino alcohol salts.

5. Composition according to any one of the preceding claims, comprising the carboxylate anionic surfactants in an amount ranging from 1% to 8% by weight and preferentially from 1.5% to 7.5% by weight, relative to the total weight of the composition.

6. Composition according to any one of the preceding claims, also comprising one or more additional surfactants chosen from nonionic surfactants, anionic surfactants other than the carboxylate anionic surfactants, and amphoteric surfactants.

7. Composition according to any one of the preceding claims, comprising a total amount of nonionic, anionic and amphoteric surfactants ranging from 0.1% to 10% by weight, preferably ranging from 1% to 8% by weight and preferentially from 1.5% to 7.5% by weight, relative to the total weight of the composition.

8. Composition according to any one of the preceding claims, comprising a total amount of surfactants ranging from 0.1% to 10% by weight, preferably ranging from 1% to 8% by weight and preferentially from 1.5% to 7.5% by weight, relative to the total weight of the composition.

9. Composition according to any one of the preceding claims, in which the ratio (weight percentage) "nonionic surfactants + anionic surfactants + amphoteric surfactants"/"nonionic associative polymers" is less than or equal to 3; it preferably ranges from 0.01 to 3, especially from 0.01 to 2.8 and preferentially from 0.1 to 2.5.

10. Composition according to one of the preceding claims, also comprising one or more polymers, especially chosen from amphoteric and cationic polymers, and also mixtures thereof.

11. Composition according to any one of the preceding claims, comprising water at a concentration preferably ranging from 5% to 98% by weight, especially from 20% to 95% by weight and better still from 50% to 90% by weight, relative to the total weight of the composition.

12. Cosmetic treatment process, especially for caring for and/or cleansing the hair and/or the scalp, comprising the application to the hair and/or the scalp of a cosmetic composition as defined in one of Claims 1 to 11, optionally followed by rinsing, after an optional leave-on time.

## Patentansprüche

1. Nicht färbende kosmetische Zusammensetzung, umfassend:
- ein oder mehrere assoziative nichtionische Polymere, bei denen es sich um Triblock-Polyurethanpolyether-Copolymere handelt, in denen es sich bei dem hydrophilem Block um eine Polyoxyethylenkette mit 50 bis 1000 und speziell 100 bis 300 Oxyethylengruppen handelt und die mindestens zwei kohlenwasserstoffbasierte lipophile Ketten mit 6 bis 30 Kohlenstoffatomen, die durch den hydrophilen Block getrennt sind, umfassen, wobei es sich bei den kohlenwasserstoffbasierten lipophilen Ketten um Seitenketten oder Ketten am Ende eines hydrophilen Blocks handeln kann, und
- ein oder mehrere anionische Carboxylat-Tenside, ausgewählt aus
- Acylglycinaten, Acylsarcosinaten, Acyllactylaten und Acylglutamaten, wobei die Acylgruppen vorzugsweise 14 bis 30 Kohlenstoffatome und noch besser 16 bis 22 Kohlenstoffatome umfassen; sowie den entsprechenden versalzten Formen; wobei diese Verbindungen gegebenenfalls oxyethyleniert sind und dann vorzugsweise 1 bis 50 Ethylenoxid-Einheiten und noch besser 1 bis 10 Ethylenoxid-Einheiten umfassen;
- polyoxyalkylenierten (C14-30)Alkylethercarbonsäuren, polyoxyalkylenierten (C14-30)-Alkyl(C6-30)-arylethercarbonsäuren, polyoxyalkylenierten (C14-30)Alkylamidoethercarbonsäuren und Salzen davon, insbesondere denjenigen mit 2 bis 50 Ethylenoxid-Einheiten;
wobei die Zusammensetzung assoziatives nichtionisches Polymer bzw. assoziative nichtionische Polymere in einer Menge im Bereich von 2 bis 60 Gew.-%, bezogen auf das Gesamtgewicht der Zusammensetzung, umfasst und
wobei die Zusammensetzung anionisches Carboxylat-Tensid bzw. anionische Carboxylat-Tenside in einer Menge im Bereich von 0,1 bis 10 Gew.-%, bezogen auf das Gesamtgewicht der Zusammensetzung, umfasst.

2. Zusammensetzung nach Anspruch 1, wobei die assoziativen nicht ionischen Polymere aus
- assoziativen nichtionischen Polyurethanpolyethern, die durch Polykondensation von mindestens drei Verbindungen, umfassend (i) mindestens ein Polyethylenglykol mit 100 bis 180 mol Ethylenoxid, (ii) einen polyoxyethylenierten Stearylalkohol mit 100 mol Ethylenoxid und (iii) ein Diisocyanat, erhältlich sind;
- assoziativen nichtionischen Polyurethanpolyethern, die durch Polykondensation von mindestens drei Verbindungen, umfassend (i) mindestens ein Polyethylenglykol mit 150 bis 180 mol Ethylenoxid, (ii) Stearylalkohol oder Decylalkohol und (iii) mindestens ein Diisocyanat, erhältlich sind; ausgewählt sind.

3. Zusammensetzung nach einem der vorhergehenden Ansprüche, umfassend die assoziativen nicht ionischen Polymere in einer Menge im Bereich von 2,5 bis 40 Gew.-%, noch besser 2,7 bis 20 Gew.-% oder sogar 2,75 bis 15 Gew.-%, bezogen auf das Gesamtgewicht der Zusammensetzung.

4. Zusammensetzung nach einem der vorhergehenden Ansprüche, wobei die anionischen Carboxylat-Tenside aus (C14-C30)Acylglutamaten und insbesondere Stearoylglutamaten, Lauroylglutamaten und Cocoylglutamaten; (C14-C30)Acylsarkosinaten und insbesondere Palmitoylsarcosinaten, Stearoylsarcosinaten, Lauroylsarcosinaten und Cocoylsarcosinaten; (C14-C30)Acyllactylaten und insbesondere Behenoyllactylaten, Lauroyllactylaten und (Iso)stearoyllactylaten; Alkylethercarboxylaten und insbesondere Laurylethercarboxylaten und Mischungen davon, insbesondere in der Form von Alkalimetall-, Erdalkalimetall-, Ammonium-, Amin- oder Aminoalkoholsalzen, ausgewählt sind.

5. Zusammensetzung nach einem der vorhergehenden Ansprüche, umfassend die anionischen Carboxylat-Tenside in einer Menge im Bereich von 1 bis 8 Gew.-% und bevorzugt von 1,5 bis 7,5 Gew.-%, bezogen auf das Gesamtgewicht der Zusammensetzung.

6. Zusammensetzung nach einem der vorhergehenden Ansprüche, ebenfalls umfassend ein oder mehrere zusätzliche Tenside, die aus nichtionischen Tensiden, anionischen Tensiden, die von den anionischen Carboxylat-Tensiden verschieden sind, und amphoteren Tensiden ausgewählt sind.

7. Zusammensetzung nach einem der vorhergehenden Ansprüche, umfassend eine Gesamtmenge von nichtionischen, anionischen und amphoteren Tensiden im Bereich von 0,1 bis 10 Gew.-%, vorzugsweise im Bereich von 1 bis 8 Gew.-% und bevorzugt von 1,5 bis 7,5 Gew.-%, bezogen auf das Gesamtgewicht der Zusammensetzung.

8. Zusammensetzung nach einem der vorhergehenden Ansprüche, umfassend eine besamtmenge von Tensiden im Bereich von 0,1 bis 10 Gew.-%, vorzugsweise im Bereich von 1 bis 8 Gew.-% und bevorzugt von 1,5 bis 7,5 Gew.-%, bezogen auf das Gesamtgewicht der Zusammensetzung.

9. Zusammensetzung nach einem der vorhergehenden Ansprüche, wobei das Verhältnis (Gewichtsprozent) "nichtionische Tenside + anionische Tenside + amphotere Tenside"/"nichtionische assoziative Polymere" kleiner oder gleich 3 ist und vorzugsweise im Bereich von 0,01 bis 3, speziell von 0,01 bis 2,8 und bevorzugt von 0,1 bis 2,5 liegt.

10. Zusammensetzung nach einem der vorhergehenden Ansprüche, ebenfalls umfassend ein oder mehrere Polymere, die speziell aus amphoteren und kationischen Polymeren sowie Mischungen davon ausgewählt sind.

11. Zusammensetzung nach einem der vorhergehenden Ansprüche, umfassend Wasser in einer Konzentration, die vorzugsweise im Bereich von 5 bis 98 Gew.-%, speziell von 20 bis 95 Gew.-% und noch besser von 50 bis 90 Gew.-%, bezogen auf das Gesamtgewicht der Zusammensetzung, liegt.

12. Verfahren zu kosmetischen Behandlung, insbesondere zur Pflege und/oder Reinigung des Haars und/oder der Kopfhaut, bei dem man auf das Haar und/oder die Kopfhaut eine kosmetische Zusammensetzung gemäß einem der Ansprüche 1 bis 11 aufbringt und gegebenenfalls nach einer fakultativen Einwirkungszeit spült.

## Revendications

1. Composition cosmétique non colorante comprenant :
- un ou plusieurs polymères non ioniques associatifs qui sont des polyuréthanes polyéthers copolymères triblocs dont la séquence hydrophile est une chaîne polyoxyéthylénée comportant de 50 à 1000, notamment de 100 à 300, groupements oxyéthylénés; et comportant au moins deux chaînes lipophiles hydrocarbonées ayant de 6 à 30 atomes de carbone, séparées par ladite séquence hydrophile, lesdites chaînes lipophiles hydrocarbonées pouvant être des chaînes pendantes ou des chaînes en bout de séquence hydrophile; et
- un ou plusieurs tensioactifs anioniques carboxylates, choisis parmi :
- les acylglycinates, les acylsarcosinates, les acyllactylates et les acylglutamates, les groupes acyle comportant de préférence de 14 à 30 atomes de carbone, mieux de 16 à 22 atomes de carbone; ainsi que les formes salifiées correspondantes; ces composés pouvant être optionnellement oxyéthylénés et comportant alors de préférence de 1 à 50 motifs oxyde d'éthylène, mieux de 1 à 10 motifs oxyde d'éthylène;
- les acides (alkyl en C14-30)éther-carboxyliques polyoxyalkylénés, les acides (alkyl en C14-30) (aryl en C6-30)éther-carboxyliques polyoxyalkylénés, les acides (alkyl en C14-30)amidoéther-carboxyliques polyoxyalkylénés et leurs sels, en particulier ceux comportant de 2 à 50 motifs oxyde d'éthylène ;
la composition comprenant 2 à 60% en poids par rapport au poids total de la composition, d'un ou plusieurs polymères non ioniques associatifs, et
la composition comprenant un ou plusieurs tensioactifs anioniques carboxylates en une quantité allant de 0,1% à 10% en poids, par rapport au poids total de la composition.

2. Composition selon la revendication 1, dans laquelle les polymères non ioniques associatifs sont choisis parmi :
- les polyuréthanes polyéthers non ioniques associatifs susceptibles d'être obtenus par polycondensation d'au moins trois composés comprenant (i) au moins un polyéthylèneglycol comprenant de 100 à 180 moles d'oxyde d'éthylène, (ii) un alcool stéarylique polyoxyéthyléné comprenant 100 moles d'oxyde d'éthylène et (iii) un diisocyanate ;
- les polyuréthanes polyéthers non ioniques associatifs susceptibles d'être obtenus par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

3. Composition selon l'une quelconque des revendications précédentes, comprenant les polymères non ioniques associatifs en une quantité allant de 2,5 à 40% en poids, encore mieux de 2,7 à 20% en poids, voire de 2,75 à 15% en poids, par rapport au poids total de la composition.

4. Composition selon l'une quelconque des revendications précédentes, dans laquelle les tensioactifs anioniques carboxylates sont choisis parmi les acyl(C14-C30)glutamates et en particulier les stéaroylglutamates, les lauroylglutamates et les cocoylglutamates; les acyl(C14-C30)sarcosinates et en particulier les palmi-toylsarcosinates, les stéaroylsarcosinates, les lauroylsarcosinates et les co-coylsarcosinates; les acyl(C14-C30)lactylates et en particulier les béhénoyllacty-lates, les lauroyllactylates et les (iso)stéaroyllactylates; les alkyl éther carboxylates et en particulier les lauryléthercarboxylates; et leurs mélanges, en particulier sous forme de sels de métaux alcalins ou alcalino-terreux, d'ammonium, d'amine ou d'aminoalcool.

5. Composition selon l'une quelconque des revendications précédentes, comprenant les tensioactifs anioniques carboxylates en une quantité allant de 1 à 8% en poids, préférentiellement de 1,5 à 7,5% en poids, par rapport au poids total de la composition.

6. Composition selon l'une quelconque des revendications précédentes, comprenant en outre un ou plusieurs tensioactifs additionnels choisis parmi les tensioactifs non ioniques, les tensioactifs anioniques autres que les tensioactifs anioniques carboxylates et les tensioactifs amphotères.

7. Composition selon l'une quelconque des revendications précédentes, comprenant une quantité totale de tensioactifs non ioniques, anioniques et amphotères, allant de 0,1 à 10% en poids, de préférence allant de 1 à 8% en poids, préférentiellement de 1,5 à 7,5% en poids, par rapport au poids total de la composition.

8. Composition selon l'une quelconque des revendications précédentes, comprenant une quantité totale de tensioactifs allant de 0,1 à 10% en poids, de préférence allant de 1 à 8% en poids, préférentiellement de 1,5 à 7,5% en poids, par rapport au poids total de la composition.

9. Composition selon l'une quelconque des revendications précédentes, dans laquelle le ratio (% en poids) "tensioactifs non ioniques + tensioactifs anioniques + tensioactifs amphotères" / "polymères associatifs non ioniques" est inférieur ou égal à 3; de préférence varie de 0,01 à 3, notamment de 0,01 à 2,8, préférentiellement de 0,1 à 2,5.

10. Composition selon l'une quelconque des revendications précédentes, comprenant en outre un ou plusieurs polymères, notamment choisis parmi les polymères amphotères et cationiques, ainsi que leurs mélanges.

11. Composition selon l'une quelconque des revendications précédentes, comprenant de l'eau à une concentration allant de préférence de 5 à 98% en poids, notamment de 20 à 95% en poids, mieux de 50 à 90% en poids, par rapport au poids total de la composition.

12. Procédé de traitement cosmétique, notamment de soin et/ou de nettoyage, des cheveux et/ou du cuir chevelu, comprenant l'application sur les cheveux et/ou le cuir chevelu, d'une composition cosmétique telle que définie à l'une des revendications 1 à 11, suivie éventuellement d'un rinçage, après un éventuel temps de pose.
